# DEMANDE DE BREVET EUROPEEN

(11) **EP 3 332 792 A1**
(43) Date de publication de la demande: **13.06.2018**
(21) Numéro de dépôt: 16202938.3
(22) Date de dépôt: 08.12.2016
(51) Int. Cl.: A61K 36/06, A61K 8/99, A61P 9/08

(54) **UTILISATION D'UN PRODUIT DE DEGORGEMENT DE VIN MOUSSEUX COMME VASODILATATEUR**

(71) Demandeur: Bochenek, Stephane, 91330 Yerres (FR)
(72) Inventeur: Bochenek, Stephane, 91330 Yerres (FR)
(74) Mandataire: Vialle-Presles, Marie José

(57) **Abrégé**

L'invention concerne l'utilisation de produit de dégorgement de vin mousseux pour l'obtention de vasodilatateurs, ainsi que des compositions thérapeutiques ou cosmétiques obtenues à partir dudit produit.

## Description

L'invention est relative à l'utilisation de produit de dégorgement de vin mousseux pour l'obtention de vasodilatateurs.

Le dégorgement est une étape spécifique de la fabrication des vins mousseux par la méthode traditionnelle (également dénommée méthode champenoise dans le cas des vins de Champagne).

Cette méthode implique en effet deux fermentations successives. La première fermentation, qui est effectuée en cuves ou en foudres, produit un vin de base tranquille. Ce vin de base est ensuite additionné d'une « liqueur de tirage » contenant du sucre et de nouvelles levures, et mis en bouteilles où se produira la seconde fermentation, appelée « prise de mousse ». C'est le CO₂ produit lors de cette fermentation en bouteille qui confère au vin mousseux son effervescence. Lorsque tout le sucre a été consommé par les levures, celles-ci meurent progressivement, formant un dépôt de lies constitué essentiellement de cellules mortes et de débris cellulaires résultant de leur autolyse. Ce dépôt de lies, qui a préalablement été concentré dans le goulot de la bouteille par une opération dénommée : « remuage », est éliminé par l'opération de dégorgement. Généralement, préalablement au remuage et au dégorgement, le vin subit une étape de « maturation sur lies » (également parfois dénommée « maturation sur lattes ») au cours de laquelle il est conservé en bouteilles au contact des lies pendant une durée variant de quelques mois à plusieurs années.

Le dégorgement peut s'effectuer « à la volée »: la bouteille est tenue tête en bas, puis redressée prestement et décapsulée simultanément pour entrainer l'expulsion du dépôt de lies sous l'effet de la pression qui règne à l'intérieur de la bouteille. Cette technique est toutefois très délicate à mettre en oeuvre et l'on utilise actuellement le plus souvent la méthode du dégorgement « à la glace » : l'extrémité du goulot où se trouve le dépôt est plongée dans un bain réfrigéré, pour congeler le dépôt. Puis la capsule est enlevée et le glaçon formé est expulsé par la pression.

Quelle que soit la méthode utilisée, les produits de dégorgement sont habituellement collectés en vue de leur destruction.

Il a maintenant été découvert que ces produits de dégorgement de vins mousseux préparés selon la méthode traditionnelle contenaient un ou plusieurs principe(s) actif(s) capable(s) d'induire un effet vasodilatateur se manifestant notamment par la capacité à inhiber la vasoconstriction induite par la phényléphrine. Cet effet vasodilatateur apparaît médié par l'endothélium vasculaire et dépendant de la production de monoxyde d'azote (NO) dans celui-ci. La présente invention résulte de cette découverte.

La présente invention a en conséquence pour objet un produit de dégorgement de vin mousseux ou une fraction active dudit produit pour l'utilisation comme médicament, notamment comme médicament vasodilatateur. Ledit médicament peut être utilisé notamment dans le cadre de la prévention ou du traitement d'états pathologiques associés à une vasoconstriction indésirable.

La présente invention a en outre pour objet l'utilisation cosmétique d'un produit de dégorgement de vin mousseux ou d'une fraction active dudit produit. Il peut s'agir notamment d'une utilisation dans un traitement non-thérapeutique visant à améliorer l'état et/ou l'aspect de la peau, des muqueuses ou des phanères par une stimulation locale de la microcirculation.

La présente invention a également pour objet des compositions pharmaceutiques ou cosmétiques comprenant en tant que principe actif, un produit de dégorgement de vin mousseux ou une fraction active dudit produit.

La présente invention a aussi pour objet l'utilisation d'un produit de dégorgement de vin mousseux, ou d'une fraction active dudit produit, comme matière première pour la préparation d'un ou plusieurs principe(s) actif(s) utilisables dans un but thérapeutique ou cosmétique. Il s'agit notamment de principe(s) actifs ayant un effet vasodilatateur tel que décrit ci-dessus.

Dans ce cadre, la présente invention a pour objet un procédé de préparation d'un principe actif ou d'un mélange de principes actifs ayant un effet vasodilatateur, caractérisé en ce qu'il comprend la purification, par tous moyens appropriés, connus en eux-mêmes, dudit principe actif ou dudit mélange à partir d'un produit de dégorgement de vin mousseux, ou d'une fraction active dudit produit.

On entend ici par « produit de dégorgement de vin mousseux » tout produit susceptible d'être obtenu par une opération de dégorgement telle que définie ci-dessus, effectuée après la prise de mousse d'un vin mousseux. De préférence, ladite opération de dégorgement est effectuée après une étape de maturation sur lies d'une durée d'au moins 6 mois, de préférence au moins 9 mois, et de manière tout à fait préférée d'au moins 12 mois, et pouvant aller jusqu'à 10 ans, de préférence jusqu'à 5 ans, et de manière tout à fait préférée jusqu'à 3 ans.

Le vin mousseux à partir duquel est obtenu ce produit de dégorgement est un vin mousseux préparé par la méthode traditionnelle. De préférence il s'agit d'un vin blanc mousseux et de manière tout à fait préférée d'un vin de Champagne.

On entend ici par « fraction active » du produit de dégorgement toute fraction dudit produit pouvant être obtenue à partir de celui-ci par une technique de séparation telle que la décantation, la centrifugation, la filtration, etc., et possédant une activité vasodilatatrice. La présence de cette activité peut aisément être vérifiée par l'homme du métier par des tests simples, tels que ceux décrits dans les exemples ci-après. Notamment, ladite fraction active peut être un surnageant de centrifugation ou de décantation du produit de dégorgement.

Ledit produit de dégorgement ou ladite fraction active peuvent être utilisés tels quels. Ils peuvent également être concentrés au préalable, notamment par déshydratation et de préférence par lyophilisation.

Les compositions pharmaceutiques ou cosmétiques selon la présente invention peuvent se présenter sous une forme adaptée à l'utilisation par voie locale au niveau de la peau ou des muqueuses notamment dans le cas des compositions cosmétiques, ou par voie générale, par exemple orale ou parentérale, principalement dans le cas des compositions pharmaceutiques.

Dans les compositions pharmaceutiques ou cosmétiques conformes à l'invention, le produit de dégorgement de vin mousseux ou sa fraction active sont associés à un milieu pharmacologiquement ou cosmétiquement acceptable et comprenant les excipients appropriés, nécessaires à la formulation. Ces compositions peuvent comprendre également le cas échéant, un ou plusieurs autres principes actifs. Le choix de ces différents constituants sera effectué par l'homme du métier en fonction du type d'utilisation, et de la voie d'administration envisagés.

La présente invention sera mieux comprise à l'aide du complément de description qui va suivre qui se réfère à des exemples non-limitatifs illustrant les propriétés vasodilatatrices d'un produit de dégorgement de vin mousseux.

### LEGENDE DES FIGURES

Figure 1 : Représentation du produit de dégorgement après décantation
Figure 2 : Enregistrements en temps réel des effets du produit de dégorgement sur des aortes de souris pré-contractées à la phényléphrine (Phe). Les flèches indiquent le moment de l'injection du produit de dégorgement L'arrêt de l'expérimentation est précisé par les lavages.
Figure 3 : Enregistrements en temps réel des effets des solutions #1 et #2 sur des aortes de souris pré-contractées à la phényléphrine (Phe). Les flèches indiquent le moment de l'injection des solutions #1 ou #2. L'arrêt de l'expérimentation est précisé par les lavages.
Figure 4 : Enregistrements en temps réel des effets de la solution #1 sur des aortes de souris traitées pendant 1 heure avec 10⁻⁵ M de L-NAME, puis pré-contractées à la phényléphrine (Phe). Les flèches indiquent le moment de l'injection de la solution #1. L'arrêt de l'expérimentation est précisé par les lavages.

### EXEMPLES

### Matériels et méthodes

### Produits étudiés :

Le produit de dégorgement utilisé est issu du mélange de deux cuvées de vin de Champagne, ayant respectivement 2 et 3 ans de maturation sur lies en bouteille avant l'étape de dégorgement.

Le produit de dégorgement est stocké à température ambiante dans un récipient clos.

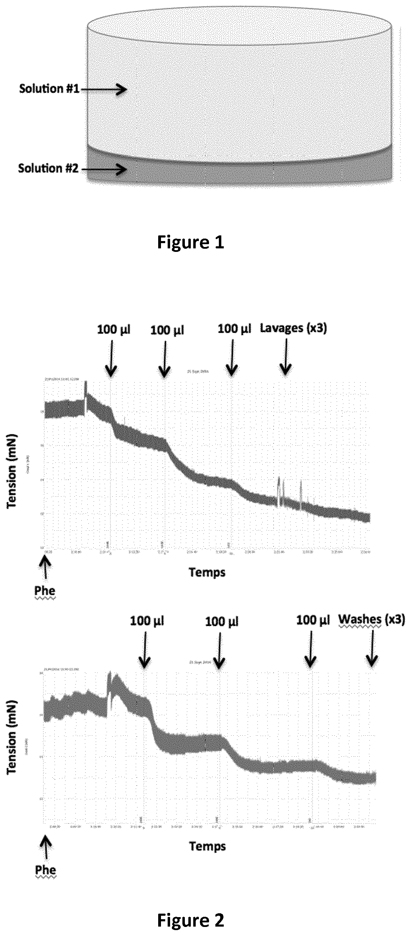

Pour certaines expérimentations le produit de dégorgement est soumis à un protocole de décantation ; à partir d'environ 2 heures de décantation on observe la séparation en deux parties distinctes (Figure 1) : la partie supérieure (dénommée ci-après solution #1) se présente sous forme d'un liquide légèrement trouble d'aspect homogène, et la partie inférieure (dénommée ci-après solution #2) se présente comme un liquide opaque contenant en suspension les débris cellulaires de levures utilisées pour la fermentation.

### Etudes de réactivité vasculaire et bronchique.

Ces études sont réalisées dans des cuves à organe isolé pour étudier la motricité des vaisseaux en réponse à différentes substances actives comme décrit précédemment (1). Brièvement, après sacrifice des souris (C57/bl6), les aortes et bronches sont prélevées et nettoyées de leur tissu conjonctif et adipeux dans une solution physiologique saline de Krebs-Henseleit (NaCl 118 mM, KCI 4.7 mM, KH₂PO₄ 1.2 mM, MgSO₄ 1.2 mM, NaSO₄ 1.2 mM, Glucose 11 mM, NaHCO₃ 25 mM et CaCl₂ 2 mM). Les aortes et bronches sont ensuite sectionnées en anneaux de 2mm de long qui sont placés individuellement dans les cuves d'un myographe de Mulvany. Chaque cuve contient 5 mL de solution de Krebs-Henseleit oxygénée et thermostatée à 37°C. Les tissus d'intérêt sont reliés à un capteur de force soumis à une pré-tension de 90 mmHg pour les aortes et de 0,5 mN pour les bronches.

Après le montage des organes, la réactivité et la viabilité des tissus sont évaluées en réalisant une stimulation des tissus avec 90 mM de chlorure de potassium (KCI). Cette étape est réalisée 3 fois avant de commencer le protocole expérimental. Si la réponse au KCI est inférieur à 1 mM, les organes sont considérés « endommagés » et sont exclus de la suite du protocole.

Les propriétés motrices sont ensuite testées en réponse à la phényléphrine (Phe), un analogue de l'adrénaline, dans le cas des aortes, et au carbachol (Cch), un analogue de l'acétylcholine, dans le cas des bronches. Les enregistrements sont analysés grâce au logiciel LabChart.

Pour tester la voie du monoxyde d'azote (NO), les aortes sont traitées 1h avec 10⁻⁵M de L-NAME (Sigma Aldrich) afin d'inhiber l'enzyme responsable de la synthèse du NO, la eNOS.

### RESULTATS

### Etudes de réactivité vasculaire.

Les propriétés vasoactives du produit de dégorgement de Champagne ont dans un premier temps été testées, en l'absence de pré-stimulation, sur les aortes pré-tendues à 90 mmHg. L'ajout de 100 à 1000 µl de ce produit ne modifie pas l'état contractile des aortes (résultats non montrés).

Les aortes ont été ensuite préalablement stimulées avec 10⁻⁵M de phényléphrine pour induire une vasoconstriction. Lorsque la contraction induite par la phényléphrine est stable, des doses successives de 100 µl de produit de dégorgement sont administrées dans la cuve à organe isolé, à intervalles de 2 minutes. Les résultats sont illustrés par la Figure 2. Ces résultats montrent que le produit de dégorgement provoque une vasorelaxation dépendante de la dose d'injection et inhibe complètement la contraction induite par la phényléphrine.

Afin de vérifier que la vasodilatation observée n'est pas induite par une cytotoxicité du produit de dégorgement, les aortes ont ensuite été lavées 3 fois avec de la solution physiologique de Krebs et restimulées avec la même concentration de phényléphrine. La contraction générée dans ces conditions est la même que celle observée avant les injections du produit de dégorgement. Ces résultats indiquent qu'un principe actif est présent dans le produit de dégorgement, et permet d'induire une vasodilatation sans toxicité cellulaire.

Les mêmes expérimentations ont été effectuées en remplaçant le produit de dégorgement par du vin de Champagne. Aucun effet vasodilatateur n'a été observé (résultats non montrés). Ces résultats montrent la présence spécifique dans le produit de dégorgement d'un ou plusieurs principes actifs vasodilatateurs absents du Champagne.

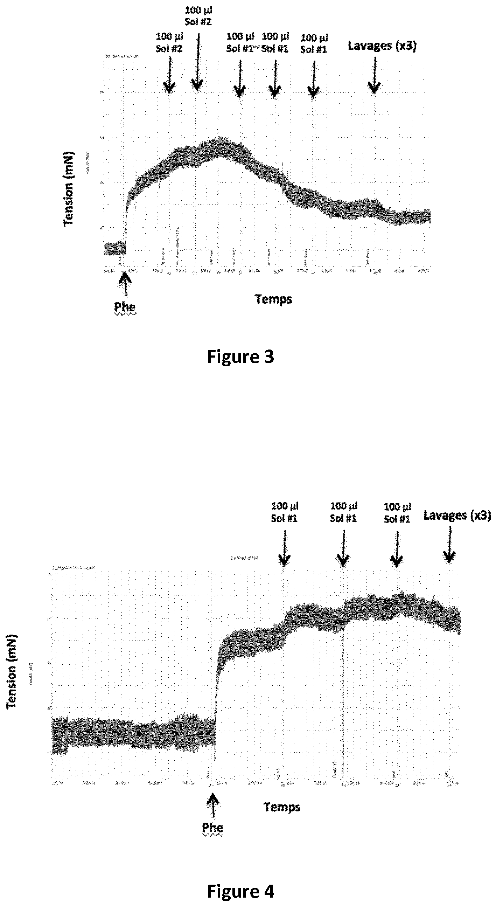

Le même protocole a également été utilisé afin de déterminer si le principe actif se retrouvait dans l'une et/ou l'autre des solutions récupérées après décantation (solution #1 ou solution #2) du produit de dégorgement. Les résultats sont montrés sur la figure 3. Alors que la solution #2 n'induit aucun effet sur le tonus vasculaire, la solution #1 produit les mêmes effets que le produit de dégorgement.

### Spécificité d'action

Afin de déterminer si les effets observés avec le produit de dégorgement et la solution #1 résultaient ou non d'une action directe sur les cellules musculaires lisses vasculaires, ces produits ont été testés sur un autre organe riche en cellules musculaires lisses : les bronches. La contractilité bronchique a été étudiée selon le même protocole que celui utilisé pour l'étude de la vasomotricité, à la différence près que la bronchoconstriction a été induite par le carbachol.

Les injections successives du produit de dégorgement ou de la solution #1 n'ont eu aucun effet sur l'état de contraction des bronches (résultats non montrés).

L'ensemble de ces résultats montre que le produit de dégorgement et la solution #1 contiennent un principe actif permettant d'induire la relaxation des artères sans agir directement sur les cellules musculaires lisses.

### Action de la solution #1 sur la voie de signalisation intracellulaire dépendante du monoxyde d'azote.

Les vaisseaux sont composés de cellules musculaires lisses, mais également de cellules endothéliales. Ce dernier type cellulaire participe activement au contrôle du tonus vasculaire et ainsi à l'état de contraction des cellules musculaires lisses (2) par la sécrétion de vasoconstricteurs et de vasodilatateurs. Parmi ces derniers, le plus puissant est le monoxyde d'azote ou NO qui est un gaz synthétisé dans les cellules endothéliales grâce à l'enzyme eNOS (endothelium Nitric Oxide Synthase). Le NO diffuse dans les cellules musculaires lisses vasculaires et active la synthèse de cGMP qui permet l'activation d'une sérine/thréonine kinase dépendante du cGMP, la PKG. Cette kinase permet d'induire la relaxation des cellules musculaires lisses en diminuant la concentration calcique intracellulaire et en inhibant la voie de sensibilisation au Ca²⁺ de l'appareil contractile (2-4).

Les premiers résultats obtenus suggèrent que le produit de dégorgement et la solution #1 pourraient agir sur la voie de synthèse du NO dans les cellules endothéliales. Pour vérifier cette hypothèse, les aortes ont été traitées pendant une heure avec un inhibiteur de la eNOS : le L-NAME (10⁻⁵M) (1), et les effets de la solution #1 sur la réactivité vasculaire ont été testés, en utilisant le protocole décrit plus haut. Les résultats sont montrés sur la figure 4. Après traitement au L-NAME, on n'observe plus d'effets vasodilatateurs de la solution #1.

Ces résultats démontrent que le produit de dégorgement et son surnageant de décantation (solution #1) contiennent une ou plusieurs molécules permettant la stimulation de la voie du NO pour induire ces propriétés vasodilatatrices.

### BIBLIOGRAPHIE

1. Andre G, Sandoval JE, Retailleau K, Loufrani L, Toumaniantz G, Offermanns S, Rolli-Derkinderen M, Loirand G, Sauzeau V. Smooth muscle specific Rac1 deficiency induces hypertension by preventing p116RIP3-dependent RhoA inhibition. J Am Heart Assoc 2014; 3: e000852.
2. Loirand G, Guerin P, Pacaud P. Rho kinases in cardiovascular physiology and pathophysiology. Circ Res 2006; 98: 322-334.
3. Loirand G, Sauzeau V, Pacaud P. Small G proteins in the cardiovascular system: physiological and pathological aspects. Physiol Rev 2013; 93: 1659-1720.
4. Sauzeau V, Le Jeune H, Cario-Toumaniantz C, Smolenski A, Lohmann SM, Bertoglio J, Chardin P, Pacaud P, Loirand G. Cyclic GMP-dependent protein kinase signaling pathway inhibits RhoA-induced Ca2+ sensitization of contraction in vascular smooth muscle. J Biol Chem 2000; 275: 21722-21729.

## Revendications

1. Produit de dégorgement d'un vin mousseux ou fraction active dudit produit pour l'utilisation comme médicament.

2. Produit de dégorgement d'un vin mousseux ou fraction active dudit produit pour l'utilisation selon la revendication 1, **caractérisé en ce que** ledit médicament a un effet vasodilatateur.

3. Produit de dégorgement d'un vin mousseux ou fraction active dudit produit pour l'utilisation selon la revendication 1, **caractérisé en ce que** ledit médicament induit une stimulation de la microcirculation.

4. Utilisation non-thérapeutique d'un produit de dégorgement d'un vin mousseux ou d'une fraction active dudit produit, dans un traitement cosmétique.

5. Utilisation selon la revendication 4, **caractérisée en ce que** ledit traitement vise à améliorer l'état et/ou l'aspect de la peau, des muqueuses ou des phanères par une activation locale de la microcirculation.

6. Composition pharmaceutique ou cosmétique comprenant en tant que principe actif, un produit de dégorgement de vin mousseux ou une fraction active dudit produit.

7. Utilisation d'un produit de dégorgement de vin mousseux, ou d'une fraction active dudit produit, comme matière première pour la préparation d'un ou plusieurs principe(s) actif(s) utilisables dans un but thérapeutique ou cosmétique.

8. Procédé de préparation d'un principe actif ou d'un mélange de principes actifs ayant un effet vasodilatateur, **caractérisé en ce qu'**il comprend la purification, par tous moyens appropriés, dudit principe actif ou dudit mélange à partir d'un produit de dégorgement de vin mousseux, ou d'une fraction active dudit produit.
